# EUROPEAN PATENT SPECIFICATION

(11) **EP 0 605 219 B1**
(45) Date of publication and mention of the grant of the patent: **22.04.1998**
(21) Application number: 93310512.4
(22) Date of filing: 23.12.1993
(51) Int. Cl.: C12N 7/04, A23J 3/08

(54) **Products containing active milk protein components and process for producing the same**
Aktive Milchproteinkomponent enthaltende Produkten, und Verfahren zu ihrer Herstellung
Produits contenants des protéins de lait comme constiuants actifs, et procédé pour préparer ces produits

(30) Priority: 25.12.1992 JP 358929/92
(43) Date of publication of application: 06.07.1994
(73) Proprietor: JCR PHARMACEUTICALS Co., LTD., Ashiya, Hyogo 659 (JP)
(72) Inventor: Yuki, Yoshikazu, Kobe, Hyogo 651-22 (JP); Terasawa, Kumiko, Nishi-ku, Kobe, Hyogo 651-21 (JP); Nakagawa, Tomoyo, Kobe, Hyogo 655 (JP); Kato, Kazuo, Kobe, Hyogo 655 (JP)
(74) Representative: Burford, Anthony Frederick

(56) References cited:
- EP-A- 0 035 204
- EP-A- 0 077 870
- EP-A- 0 117 064
- EP-A- 0 479 597
- FR-A- 1 444 725
- GB-A- 585 586
- DATABASE WPI Week 8306, Derwent Publications Ltd., London, GB; AN 83-13830 & JP-A-57 212 194 (EIKEN KAGAKU KK) 27 December 1982
- PATENT ABSTRACTS OF JAPAN vol. 009, no. 059 (C-270)15 March 1985 & JP-A-59 196 820 (NIHON CHEMICAL RESEARCH KK) 8 November 1984

## Description

This invention relates to virus-free products containing active milk components produced by heating, for example, human mother's milk (hereinafter referred to briefly as "mother's milk") or other kinds of milk under such conditions as preserve activities of the useful, active protein components present therein, such as immunoglobulins, lactoferrin and lysozyme, without causing any deterioration thereof, and to a process for producing the same.

Though progress in premature infant therapy has made it possible to improve markedly the survival rates of immature and premature infants, problems still have been left unsolved regarding how premature infants should be nourished. Premature infants are presently nourished mainly by means of mother's milk, but, with their inadequate nutrition accumulation and rapid growth, they tend to suffer from serious lack of nourishment principally in terms of protein and minerals, resulting in considerable concern about eventual adverse effects on their subsequent growth and development. As a means of supplementing the nutritional elements, attempts have been made, mainly in Europe, to utilize fortified mother's milk in which the nutritional elements deficient in mother's milk are supplemented [Goldblum, R.M. et al., Pediatric Research, 25, 184 (1989), Hagelberg, S. et al., Acta Pediatr. Scand., 79, 1163 (1990)]. Fortifying substances for mother's milk include vitamins, minerals and sugars as well as protein components contained in cow's milk or mother's milk.

Fortification of mother's milk is considered of significance from the standpoints of complete nutrition and conferment of immunity, and it was reported that fortified mother's milk was prepared on a laboratory scale for testing purpose and, when given to premature or immature infants, increased body weights, suppressed diarrhoea or lowered the incidence of necrotic colitis [Howie, P.W. et al., Br. Med. J., 300, 11 (1990); Moro, G. E. et al., J. Pediatr. Gastroenterol. Nutr., 13, 150 (1991); Lucas, A. et al., The Lancet, 336, 1519 (1990)]. In the case of mother's milk, particularly pooled mother's milk, anti-viral treatment against pollution with AIDS virus and other pathogenic viruses constitutes serious concern. The sterilization treatment of such milk components is at present carried out merely by means of heat treatment for a short period of time or anti-viral treatment of such components is conducted merely by way of inactivation treatment at 56°C for 30 min. Under the above-described conditions, AIDS virus were reported to be inactivated [Eglin, R. P. et al., The Lancet, May 9, 1093 (1987)], but densely infected AIDS virus was regarded as being far from completely inactivated [Raskin, L. et al., JAMA, 255, 1857 (1986)].

As the reliable procedure of erasing pathogenic viruses established in the light of the present standard of learning, heat treatment in the liquid state at 60°C for 10 hours (hereinafter referred to as "liquid-state heating") was adopted on the basis of the report by Murray [The New York Academy of Medicine, 31, 341 (1955)], and has since been in extensive use. However, the procedure is applied only to substances being capable of withstanding liquid-state heating, such as albumins; the procedure causes active protein components in mother's milk, such as secretory immunoglobulins, lactoferrin and lysozyme, to undergo thermal denaturation at 62.5°C for 30 min., bringing about diminished activities or complete deactivation [Evans T. J. et al., Archives of Disease in Childhood, 58, 239 (1978)]. It was demonstrated by the present Inventors that secretory immunoglobulin A as purified from mother's milk remains stable, even after being subjected to the liquid-state heating at 60°C for 10 hours [JP-A-145031/1992 (corresponding to EP-A-0479597)], but attempts so far to heat sterilize lactoferrin, lysozyme and other useful, active protein components have all been unsuccessful.

The present Inventors, have found that the active protein components contained in milk can be obtained with enhanced safety and effectiveness by liquid-state heating of defatted milk under neutral pH conditions, followed by salting-out under weakly acid conditions.

The present invention relates to products containing active milk protein components which are produced by a process comprising the steps of adding to milk plasma or milk serum a sugar alcohol or disaccharide, optionally with an amino acid, and conducting heat treatment of the resultant mixture at pH 6 to 8 preferably at about 60°C for about 10 hours and precipitating the active milk proteins by adding ammonium sulfate at a concentration of 30 to 60% (W/V) to the heat-treated mixture, and processes for producing the same.

In accordance with the present invention there is provided a process for producing a virus free product containing active milk protein components by heating of a milk fraction with a stabiliser at a temperature and for a sufficient time to inactivate pathogenic viruses, then precipitating out the active milk protein components characterised in that a stabilizer selected from sugar alcohols or disaccharides at a concentration of 30% to 70% (W/V), and mixtures of a sugar alcohol or disaccharide at said concentration with an amino acid at a concentration of 10% to 20% (W/V) is added to a milk fraction which is milk plasma or milk serum and the resultant solution heated at pH 6 to 8, and in that ammonium sulphate at a concentration of 30% to 60% (W/V) is then added to the resultant heat-treated solution at pH 4 to 7 to precipitate the active milk protein components.

In the present invention, use is made of milk plasma or milk serum from animal milk such as human milk and cow's milk. As is known well, the milk fluid is defatted to give milk plasma, which is then deprived of casein to produce milk serum.

In the present invention, milk plasma or milk serum preferably is heated at about 60°C for about 10 hours in the presence of an added stabilizing agent to inactivate viruses.

Addition of the stabilizing agent is of utmost importance not only in preventing milk fluid from becoming turbid during heating but also suppressing denaturation or deactivation of active protein components contained in milk fluid such as secretory immunoglobulin, lactoferrin and lysozyme.

The term "active protein components" as used in the present invention, designates any protein that, in minute amounts, exhibits physiological and pharmacological activities.

As the stabilizing agent, there are used sugar alcohols, such as sorbitol and mannitol, and disaccharides, such as cane sugar and malt sugar, solely or in admixture with amino acids, such as glycine and alanine.

One or more of these stabilizing agents are employed, and in cases where they are utilized alone, it is preferred to use sugar alcohols which do not cause any brown-coloration reaction, such as sorbitol.

Although the stabilizing agents are desirably used at higher concentrations, it is recommended for the purpose of increasing salting-out efficiency after heat treatment that the amino acids should be utilized at 10 to 20% (W/V), while the sugar alcohols or disaccharides are employed at 30 to 70% (W/V), preferably 40 to 70% (W/V).

In JP-A-145031/1992 (corresponding to EP-A-0479597), secretory immunoglobulin A was subjected to liquid-state heating at about 60°C for about 10 hours in the presence of the same stabilizing agent,
whereby there was no need to control the pH value of the solution. However, in liquid-state heating collectively and comprehensively the several active protein components in milk fluid, the pH value of the solution containing the stabilizing agent is of paramount importance. In the acid pH range, lactoferrin and lysozyme are stable, whereas secretory immunoglobulin A becomes turbid and undergoes polymerization, being susceptible to denaturation. In the alkaline pH range, on the other hand, lactoferrin and lysozyme become unstable. The present invention is based on the discovery that heating at a pH in the neighbourhood of neutrality (pH 6 to 8) permits these useful active protein components in raw milk to be recovered in comprehensive and stable manner.

It was demonstrated, with poliovirus type I as a model virus, that viruses are inactivated by conducting liquid-state heating of a milk fluid at a pH 7.0 and at 60°C for 10 hours in the presence of 65% of sorbitol, which conditions are encompassed by the heat treatment conditions of the present invention (Table 1). The results suggest that liquid-state heating in the presence of the stabilizer according to the present invention would result in inactivation of AIDS virus and cytomegalovirus which individually show thermal resistance inferior and equal to that of poliovirus.

The active protein components in the heat-treated milk solution are recovered by salting out with ammonium sulfate. In order to achieve this, it is recommended to use milk serum in the heat treatment because it is deprived in advance of casein. In view of the concentration of the stabilizing agent in the heat-treated milk solution, the active protein components desirably are recovered through precipitation by diluting the heat-treated milk solution with equal or double volume of water to lower its viscosity. In this manner, the recovery procedure, when done in acid pH range (pH 4 to 7), can realize the best recovery rate of the active protein components.

Precipitation with polyethylene glycol, as employed by the present Inventors in JP-A-145031/1992 (corresponding to EP-A-0479597) to recover immunoglobulins, can permit the recovery of high-molecular-weight secretory immunoglobulin A and lactoferrin from mother's milk, but fails to recover lysozyme and other components which have lower molecular weights (see Table 2).

The heat-treated milk serum of the present invention, when containing an appropriate type of the stabilizing agent at a suitable concentration, can be fed, as such or in mixture with water or other foods, as a virus-free food. The active protein components as fractionated from the heat-treated milk serum can be desalted by means of dialysis or ultrafiltration, sterilized and fed in liquid form, or after being admixed with lyophilized mother's milk or milk preparations, for premature infants or used in normal powdered milk.

Milk or other foods, which are admixed with the mother's milk components of the present invention containing only the active protein components inclusive of immunoglobulins, can be utilized as a supplement for mother's milk and a nutrition aid for premature infants as well as in the prevention of infection with viruses and in the therapy of intractable diarrhoea, and can be expected to find application not only as a food but also as a pharmaceutical.

Described below are Examples and Test Examples to illustrate this invention in more detail.

### Assay Procedures

Secretory immunoglobulin A (sIgA) was assayed in accordance with the ELISA method in which anti-secretory component antibody [produced by K.K. Igaku Seibutugaku Kenkyuusho] was utilized as a solid phase and a peroxidase-labelled anti-α-chain antibody prepared from anti-α-chain antibody [produced by Seikagaku Kogyo K.K. following Hasbida's method (J. Appl . Biochem., 6 56 (1984))] was utilized as a labelled antibody. The standard sIgA as used was prepared by the present Inventors. The virus infection value and virus neutralization antibody value were calculated on the basis of the viral cell degeneration effects found on the microplates using viral proliferated cells and established cell line MA 104 of renal origin of rhesus monkey [Uirus Jikken-gaku Soron (Studies on Virus Experiments, Elements, Maruzen Ltd. of Tokyo, Japan (1973)].

Lactoferrin was assayed in accordance with the ELISA method in which the anti-lactoferrin antibody [produced by Jackson Immunoresearch Lab.] was used as a solid phase and a peroxidase-labelled antibody [produced by Jackson Immunoresearch Lab.] was utilized as a labelled antibody. The lactoferrin manufactured by Sigma Co. was used as a standard.

The capacity of lactoferrin to combine with iron was determined in accordance with the method of Mazurier et al [Biochemica et Biophysica Acta, 629, 399 (1980)].

The lysozyme activity was assayed by the turbidity reduction method for cells of Micrococcus ruteus [Standards for Ingredients for Pharmaceuticals outside the Scope of Japanese Pharmacopoeia]. The lysozyme of egg white [manufactured by Sigma Co.] was utilized as a standard.

### Example 1:

A 5 liter portion of mother's milk was defatted by centrifugation, and the resultant supernatant liquid was adjusted to pH 4.6, followed by removal of casein by centrifugation to give a supernatant liquid (milk serum). The milk serum was adjusted to pH 7.0, admixed with sorbitol in an amount of 65% (W/V), and the resultant solution distributed into hard glass bottles, followed by air-tight closure. The glass bottles were immersed in hot water at 60°C to heat the contents for 10 hours, which were then cooled by adding a two-fold volume of cold water, adjusted to pH 5.0 and admixed with ammonium sulfate to a concentration of 50% (W/V) to conduct salting out. The precipitate fraction was collected by centrifugation and dissolved in isotonic saline, and the solution was subjected to adequate dialysis with use of a dialysis membrane with 3,000 molecular weight cut-off, followed by sterilization by means of membrane filtration. The protein recovery rate (monitored through absorbance at a wavelength of 280 nm) was 75% based on the milk serum (Lot No. HW-1). The same procedure was effected simultaneously but without heat-treatment to prepare a control (Lot No. NW-1).

### Example 2:

A 5-liter portion of human mother's milk was defatted by centrifugation, and the resultant solution (milk serum) was adjusted to pH 7.0 and admixed with sorbitol and glycine at concentrations of 50% (W/V) and 15% (V/V), respectively, followed by distribution into hard glass bottles and air-tight closure. The glass bottles were immersed into warm water at 60°C to heat the contents for 10 hours, which were then cooled by adding about 2-fold volume of water, adjusted to pH 5.0 and admixed with ammonium sulfate at a concentration of 50% (W/V) to effect salting out. The precipitate fraction was collected by centrifugation and dissolved in isotonic saline, and the resultant solution was subjected to adequate dialysis with use of a dialysis membrane with 1,000 molecular weight cutoff, followed by sterilization by means of membrane filtration. The protein recovery rate (monitored through absorbance at a wavelength of 280 nm) was 72% based on the milk serum (Lot No. HW-2). The same procedure was effected simultaneously but without heat-treatment to prepare a control (Lot No. NW-2).

### Test Example 1:

The protein components of mother's milk as prepared in Example 1 was purified through column chromatography on DEAE-Toyopearl™, an ion exchange resin, to produce secretory immunoglobulin A (sIgA). The sIgA obtained from Lot No. HW-1 was called "Lot No. H-sIgA", while the one from Lot No. NW-1 named "Lot No. N-sIgA". These test samples as well as the protein components of mother's milk as prepared in Example 1 and 2 were subjected to measurement of neutralization antibody values with Coxsackie viruses, group B type 3, group A type 4 and group A type 16. The results are shown in Table 3.

The protein components of mother's milk according to the present invention (Lot Nos. HW-1 and HW-2) and the sIgA isolated from Lot HW-1 (Lot No. H-sIgA) were observed to show the same virus neutralization antibody values with all of the Coxsackie viruses as the protein components from the mother's milk which had not been heat-treated (Lot Nos.NW-1 and NW-2) and the sIgA isolated from Lot No. NW-1 (Lot No. N-sIgA). Such results indicate that the purified sIgA's retained their virus-recognizing paratope without being denatured even after the heat treatment.

### Test Example 2:

The protein components of mother's milk as prepared in Example 1 were purified by means of affinity column chromatography on sulfurated Cellulofine to give lactoferrin. The lactoferrin purified from Lot No. HW-1 was referred to as "Lot No. H-Lf", while the one from Lot No. NW-1 called "Lot No. N-Lf". These test samples as well as the protein components of mother's milk as prepared in Examples 1 and 2 were subjected to determination of the iron combining capacity, with the result being shown in Table 4.

The mother's milk protein components of the present invention (Lot Nos. HW-1 and HW-2) as well as the lactoferrin isolated from Lot No. HW-1 (Lot No. H-Lf) were found to possess the capacity to combine with about 1.5 molecules of iron per molecule, as was the case with the protein components from mother's milk which had not been heat-treated (Lot Nos. NW-1 and NW-2) and the lactoferrin isolated from Lot No. NW-1 (Lot No. N-Lf). Since lactoferrin is known to have 2 iron combining sites in the molecule, the results reveal that the isolated lactoferrin from the protein components of mother's milk retained both iron-combining sites unchanged even after the heat treatment.

### Test Example 3:

The protein components of mother's milk as prepared in Examples 1 and 2 were subjected to measurement of the enzymatic activity of lysozyme, with the results being shown in Table 5.

The protein components of mother's milk according to the present invention (Lot Nos. HW-1 and HW-2) both were observed to show enzymatic activity of lysozyme, like the protein components of mother's milk which had not been heat-treated (Lot Nos. NW-1 and NW-2). The results indicate that the lysozyme contained in the protein components retained their substrate-combining sites and activity centers as unaffected even after heat treatment.

**Table 1:**

| | | |
|---|---|---|
| Inactivation of poliovirus through liquid-state heating with Polio virus type I as diluted with phosphate buffer PBS (pH 7.2). | | |

| Stabilizer | Liquid-state heating 60°C/10 h (TCID₅₀) | Heating 4°C/10 h (TCID₅₀) |
|---|---|---|
| None | <3.1 | 3.2 x 10⁴ |
| 65% sorbitol | <3.1 | 3.2 x 10⁴ |
| 65% sorbitol + X* | <3.1 | 5.6 x 10⁴ |

| | | |
|---|---|---|
| Note, *; 2% human serum albumin, which was added as a model protein in consideration of a possibility that the protein itself would act as a stabilizer for viruses. | | |

**Table 2:**

| | | |
|---|---|---|
| Recovery rate of mother's milk protein components | | |

| Mother's milk protein component | Salting out with (NH₄)₂SO₄* | Precipitation with polyethylene glycol* |
|---|---|---|
| sIgA | 74.2% | 65.2% |
| Lactoferrin | 70.1% | 35.7% |
| Lysozyme | 69.2% | <1% |

| | | |
|---|---|---|
| Note, *: The milk serum of the same production lot was subjected to liquid-state heating of the present invention, diluted with water and adjusted to pH 5.0, followed by division into two portions of equal volume, one of which was then salted out with 50% ammonium sulfate, with the other being subjected to recovery by precipitation with 25% polyethylene glycol. | | |

**Table 3:**

| | | | | | | |
|---|---|---|---|---|---|---|
| Virus neutralization antibody values of the mother's milk protein components and sIgA, as expressed in terms of the maximum dilution of 5 mg/cm³ of sIgA required to neutralize 100 TCID₅₀ for each virus (the concentration of sIgA was calculated based on the ELISA method). | | | | | | |

| | Mother's milk component* | | | | Purified sIgA** | |
|---|---|---|---|---|---|---|
| Virus | NW-1 | HW-1 | NW-2 | HW-2 | N-sIgA | H-sIgA |
| Coxsackie B3 | 4 | 4 | 8 | 8 | 8 | 8 |
| Coxsackie A4 | 16 | 32 | 4 | 4 | 16 | 16 |
| Coxsackie A16 | 32 | 32 | 8 | 8 | 32 | 32 |

| | | | | | | |
|---|---|---|---|---|---|---|
| Notes, *: The mother's milk components, NW-1 and HW-1 as well as NW-2 and HW-2, were prepared from the same mother's milk, with the heat treatment for HW-1 and HW-2 but without the heat treatment for NW-1 and NW-2. | | | | | | |
| **: The purified sIgA, N-sIgA and H-sIgA, were as isolated from the mother's milk components, NW-1 and HW-1. | | | | | | |

**Table 4:**

| | | | | | |
|---|---|---|---|---|---|
| The iron-combining capacity of the mother's milk components and lactoferrin, as expressed in terms of a number of the combined iron molecules | | | | | |

| Mother's milk component* | | | | Purified lactoferrin** | |
|---|---|---|---|---|---|
| NW-1 | HW-1 | NW-2 | HW-2 | N-Lf | H-Lf |
| 1.5 | 1.7 | 1.5 | 1.6 | 1.5 | 1.4 |

| | | | | | |
|---|---|---|---|---|---|
| Notes, *: The mother's milk components, NW-1 and HW-1 as well as NW-2 and HW-2, were prepared from the same mother's milk, with the heat treatment for HW-1 and HW-2 but without the heat treatment for NW-1 and NW-2. | | | | | |
| **: The purified lactoferrins, N-Lf and H-Lf, were as isolated from the mother's milk components, NW-1 and HW-1. | | | | | |

**Table 5:**

| | | | |
|---|---|---|---|
| The lysozyme activity of the mother's milk components, as calculated as converted to the quantity titer of egg-white lysozyme and as expressed in terms of the quantity titer, µg, per absorbance measured with a 10-mm long cell at a wavelength of 280 nm. | | | |

| Mother's milk component* | | | |
|---|---|---|---|
| NW-1 | HW-1 | NW-2 | HW-2 |
| 28.9 | 27.9 | 31.7 | 30.5 |

| | | | |
|---|---|---|---|
| Notes, *: The mother's milk components, NW-1 and HW-1 as well as NW-2 and HW-2, were prepared from the same mother's milk, with the heat treatment for HW-1 and HW-2 but without the heat treatment performed for NW-1 and NW-2. | | | |

## Claims

1. A process for producing a virus free product containing active milk protein components by heating of a milk fraction with a stabiliser at a temperature and for a sufficient time to inactivate pathogenic viruses, then precipitating out the active milk protein components characterised in that a stabilizer selected from sugar alcohols or disaccharides at a concentration of 30% to 70% (W/V), and mixtures of a sugar alcohol or disaccharide at said concentration with an amino acid at a concentration of 10% to 20% (W/V) is added to a milk fraction which is milk plasma or milk serum and the resultant solution heated at pH 6 to 8, and in that ammonium sulphate at a concentration of 30% to 60% (W/V) is then added to the resultant heat-treated solution at pH 4 to 7 to precipitate the active milk protein components.

2. A process as claimed in Claim 1, wherein the stabilizer is added to milk serum.

3. A process as claimed in Claims 1 or 2, wherein the stabilizer is selected from sorbitol, mannitol, cane sugar and malt sugar, optionally with alanine or glycine.

4. A process as claimed in Claim 3, wherein the stabilizer is sorbitol optionally with glycine.

5. A process as claimed in any one of the preceding claims, wherein the milk plasma or milk serum is derived from human mother's milk.

6. A process as claimed in any one of Claims 1 to 5, wherein the milk plasma or milk serum is derived from cow's milk.

7. A process as claimed in any one of the preceding claims, wherein said milk fraction is heated at about 60°C for about 10 hours.

8. A virus free precipitate derived from milk serum or milk plasma containing secretory immunoglobulin A, lactoferrin and lysozyme obtainable by a process as claimed in any one of the preceding claims.

## Patentansprüche

1. Verfahren zur Herstellung eines virusfreien Produktes mit einem Gehalt an wirksamen Milcheiweißbestandteilen durch Erhitzen einer Milchfraktion mit einem Stabilisator bei einer Temperatur und Zeitdauer, die ausreichen, pathogene Viren zu inaktivieren, wonach das Ausfällen der wirksamen Milcheiweißkomponenten erfolgt, dadurch **gekennzeichnet**, daß ein Stabilisator, der aus Zuckeralkoholen oder Disacchariden in einer Konzentration von 30 bis 70 Gew.Nol.-% und Gemischen aus einem Zuckeralkohol oder Disaccharid in besagter Konzentration mit einer Aminosäure in einer Konzentration von 10 bis 20 Gew./Vol.-% ausgewählt wird, zu einer Milchfraktion hinzugefügt wird, die Milchplasma oder Milchserum darstellt, und die so erhaltene Lösung bei einem pH-Wert von 6 bis 8 erhitzt wird, und dann Ammoniumsulfat in einer Konzentration von 30 bis 60 Gew.Nol.-% zu der so erhaltenen hitzebehandelten Lösung bei einem pH-Wert von 4 bis 7 zum Ausfällen der wirksamen Milcheiweißbestandteile hinzugegeben wird.

2. Verfahren nach Anspruch 1, bei dem der Stabilisator zu dem Milchserum hinzugefügt wird.

3. Verfahren nach den Ansprüchen 1 oder 2, bei dem der Stabilisator aus Sorbit, Mannit, Rohrzucker und Malzzucker, wahlweise zusammen mit Alanin oder Glycin ausgewählt wird.

4. Verfahren nach Anspruch 3, bei dem der Stabilisator Sorbit wahlweise zusammen mit Glycin ist.

5. Verfahren nach einem der vorstehenden Ansprüche, bei dem das Milchplasma oder Milchserum von menschlicher Muttermilch stammt.

6. Verfahren nach einem der Ansprüche 1 bis 5, bei dem das Milchplasma oder Milchserum von Kuhmilch stammt.

7. Verfahren nach einem der vorstehenden Ansprüche, bei dem die Milchfraktion bei etwa 60°C etwa 10 Stunden lang erhitzt wird.

8. Aus Milchserum oder Milchplasma stammendes virusfreies Präzipitat mit einem Gehalt an sekretorischem Immunoglobulin A, Lactoferrin und Lysozym, erhältlich mittels eines Verfahrens gemäß einem der vorstehenden Ansprüche.

## Revendications

1. Procédé de préparation d'un produit exempt de virus contenant des constituants protéiques actifs du lait, en chauffant une fraction de lait avec un stabilisant à une température et pendant un temps suffisant pour inactiver les virus pathogènes, puis en précipitant les constituants protéiques actifs du lait, caractérisé en ce qu'un stabilisant choisi parmi les itols ou les disaccharides à une concentration de 30 % à 70 % (p/v) et les mélanges d'un itol ou d'un disaccharide à ladite concentration avec un acide aminé à une concentration de 10 % à 20 % (p/v), est ajouté à une fraction de lait qui est du plasma de lait ou du sérum de lait, et la solution résultante est chauffée à un pH 6 à 8, et en ce que du sulfate d'ammonium à une concentration de 30 % à 60 % (p/v) est ensuite ajouté à la solution traitée thermiquement résultante à un pH 4 à 7 pour précipiter les constituants protéiques actifs du lait.

2. Procédé selon la revendication 1, dans lequel le stabilisant est ajouté à du sérum de lait.

3. Procédé selon les revendications 1 ou 2, dans lequel le stabilisant est choisi parmi le sorbitol, le mannitol, le saccharose et le maltose, éventuellement avec de l'alanine ou de la glycine.

4. Procédé selon la revendication 3, dans lequel le stabilisant est le sorbitol éventuellement avec de la glycine.

5. Procédé selon l'une quelconque des revendications 1 à 4, dans lequel le plasma de lait ou le sérum de lait vient de lait maternel humain.

6. Procédé selon l'une quelconque des revendications 1 à 5, dans lequel le plasma de lait ou le sérum de lait vient de lait de vache.

7. Procédé selon l'une quelconque des revendications 1 à 6, dans lequel ladite fraction de lait est chauffée à environ 60 °C pendant environ 10 heures.

8. Précipité exempt de virus venant de sérum de lait ou de plasma de lait contenant de l'immunoglobuline A sécrétoire, de la lactoferrine et du lysozyme, pouvant être obtenu par un procédé tel que revendiqué dans l'une quelconque des revendications 1 à 7.
